(19) Europäisches Patentamt
European Patent Office
Office européen des brevets

(11) Numéro de publication: **0 502 783 B1**

(12) **FASCICULE DE BREVET EUROPEEN**

(45) Date de publication de fascicule du brevet: **03.05.95**

(51) Int. Cl.[6]: **A61K 7/13**

(21) Numéro de dépôt: **92400557.2**

(22) Date de dépôt: **04.03.92**

(54) **Procédé de teinture des fibres kératiniques associant l'isatine ou ses dérivés à une aminopyridine ou aminopyrimidine, et agents de teinture.**

(30) Priorité: **05.03.91 FR 9102614**

(43) Date de publication de la demande:
**09.09.92 Bulletin 92/37**

(45) Mention de la délivrance du brevet:
**03.05.95 Bulletin 95/18**

(84) Etats contractants désignés:
**AT BE CH DE DK ES FR GB GR IT LI NL PT SE**

(56) Documents cités:
**EP-A- 0 106 987**
**EP-A- 0 359 465**
**DE-A- 2 714 831**
**DE-A- 2 716 671**

(73) Titulaire: **L'OREAL**
**14, Rue Royale**
**F-75008 Paris (FR)**

(72) Inventeur: **Lang, Gérard**
**44 avenue Lacour**
**F-95210 Saint-Gratien (FR)**
Inventeur: **Cotteret, Jean**
**15 Allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 502 783 B1

## Description

La présente invention concerne un procédé de teinture des fibres kératiniques, en particulier des cheveux humains, associant l'isatine ou l'un de ses dérivés à une aminopyridine ou aminopyrimidine, ainsi que les agents de teinture mis en oeuvre.

En coloration directe des cheveux, c'est-à-dire dans un procédé de teinture ne mettant pas en oeuvre un processus de développement des colorants par voie oxydative, on a déjà proposé d'utiliser la 2,3-indolinedione encore appelée isatine comme colorant jaune de base dans le brevet français n° 2.588.473.

La demande européenne n° 0359.465 a proposé ensuite un procédé de teinture directe utilisant l'isatine ou l'un de ses dérivés en association avec des dérivés d'aminobenzène disubstitués.

La demanderesse vient de découvrir, d'une manière surprenante, un nouveau procédé de teinture associant l'isatine ou ses dérivés à des colorants du type aminopyridine ou aminopyrimidine, permettant d'obtenir une large gamme de nuances plus résistantes aux shampooings et à la transpiration que celles obtenues avec les procédés de teinture directe utilisant les dérivés aminés connus de l'art antérieur. Les colorations obtenues sont de plus stables à la lumière, aux intempéries et aux agents chimiques.

La présente invention a donc pour objet un procédé de teinture des fibres kératiniques, consistant à appliquer sur les fibres, l'isatine ou l'un de ses dérivés, et une aminopyridine ou aminopyrimidine, soit simultanément sous forme d'un mélange extemporané, soit de façon successive.

Un objet de l'invention consiste également en un agent de teinture à deux composants.

D'autres objets apparaîtront à la lumière de la description.

Le procédé de teinture des fibres kératiniques, en particulier des cheveux humains, conforme à la présente invention, est caractérisé essentiellement par le fait qu'il comporte l'application sur lesdites fibres d'un composant (A) constitué d'une composition contenant dans un milieu approprié pour la teinture, au moins un composé de formule (I) suivante :

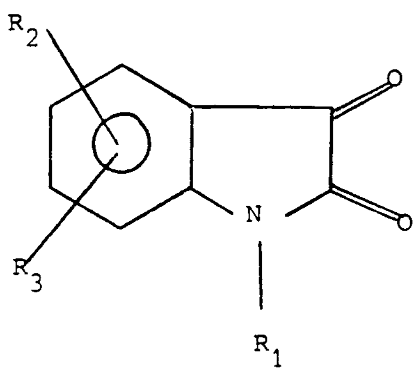

(I)

dans laquelle

$R_1$ désigne un atome d'hydrogène, un radical alkyle en $C_1$-$C_6$, acétyle, benzoyle, phényle ou carboxyalkyle en $C_1$-$C_4$;

$R_2$ et $R_3$, indépendamment l'un de l'autre, désignent un atome d'hydrogène, un alkyle en $C_1$-$C_6$, un hydroxyle, un atome d'halogène, un groupement nitro, un alkyl($C_1$-$C_6$)phényle, phényle ou un alcoxy en $C_1$-$C_4$;

et un composant (B) constitué d'une composition contenant dans un milieu approprié pour la teinture, au moins

une aminopyridine de formule (II) suivante :

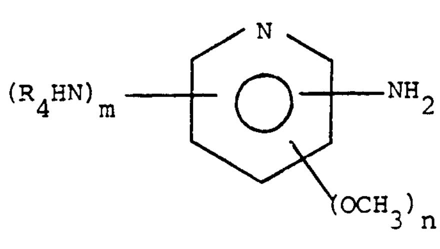

(II)

dans laquelle :

$R_4$ désigne un atome d'hydrogène ou un groupement $\beta$-hydroxy-éthyle;

n = O, 1 ou 2, et m = O ou 1;

ainsi que ses sels cosmétiquement acceptables, ou bien une aminopyrimidine de formule (III):

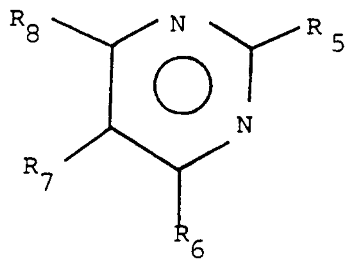

(III)

dans laquelle :

$R_5$ désigne un atome d'hydrogène, un groupement hydroxy ou un groupement

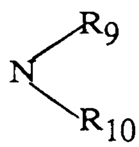

$R_6$ désigne un groupement hydroxy ou un groupement

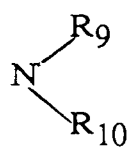

$R_7$ désigne H ou $NH_2$,

$R_8$ désigne un groupement

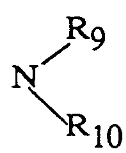

$R_9$ et $R_{10}$, indépendamment l'un de l'autre, représentant un atome d'hydrogène, un alkyle en $C_1$-$C_4$, un groupement $(CH_2)_p$-Z, où p=1 à 4 et Z représente OH, halogène, $NH_2$, NHR′, ou NHR′R″, où R′et R″ désignent un alkyle en $C_1$-$C_4$ ou forment un hétérocycle avec l'atome d'azote auxquels ils sont attachés ;

sous réserve qu'un des groupements $R_5$ à $R_8$ désigne $NH_2$;

ainsi que ses sels cosmétiquement acceptables.

Pour les formules (I), (II) et (III) ci-dessus, à titre de radicaux alkyle en $C_1$-$C_4$, on peut citer méthyle, éthyle, propyle, butyle, isopropyle, isobutyle et tert.-butyle;

les radicaux alkyle en $C_1$-$C_6$ comprennent, outre ceux cités ci-dessus, les radicaux pentyle (linéaire et ramifiés) et hexyle (linéaire et ramifiés).

A titre de radicaux alcoxy en $C_1$-$C_4$, on peut citer méthoxy, éthoxy, propoxy et butoxy.

A titre de radicaux hydroxyalkyle, on peut citer le 2-hydroxy-éthyle, les 2 ou 3-hydroxypropyle.

A titre de radicaux polyhydroxyalkyle, on peut citer le 2,3-dihydroxypropyle, le 3,4-dihydroxybutyle.

Les hétérocycles formés avec l'atome d'azote sont de préférence des cycles pipéridino, morpholino ou pipérazino.

Les sels cosmétiquement acceptables sont choisis de préférence parmi les chlorhydrates, les bromhydrates, les sulfates.

Le procédé selon l'invention peut être mis en oeuvre sans l'intervention d'un agent oxydant autre que l'air.

Le procédé de teinture décrit ci-dessus conduit à la formation d'une base de Schiff, soit lors du mélange du composant (A) avec le composant (B), soit in situ dans la fibre kératinique lors d'une application séquentielle des composants (A) et (B). Cette base de Schiff a pour formule :

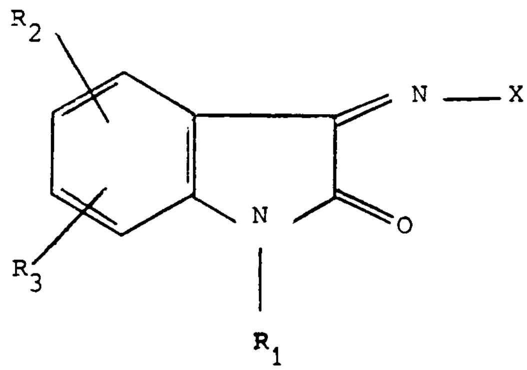

(IV)

dans laquelle X désigne :

a)

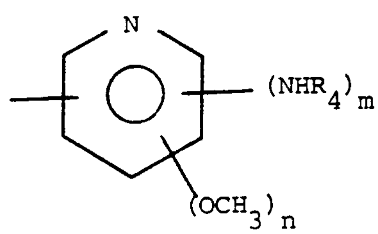

où $R_4$, m et n ont les significations ci-dessus indiquées pour la formule (II),

ou b)

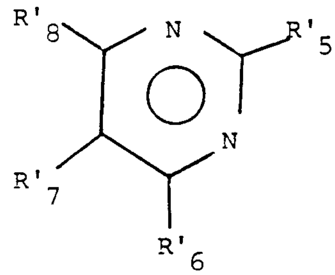

où $R'_5$ désigne H, OH,

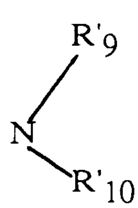

ou une liaison covalente,

$R'_6$ désigne OH,

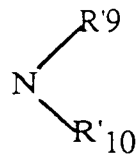

ou une liaison covalente,

$R'_7$ désigne H ou une liaison covalente, et

$R'_8$ désigne

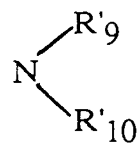

ou une liaison covalente,

$R'_9$ et $R'_{10}$ ayant les mêmes significations que $R_9$ et $R_{10}$, l'un des deux étant cependant différent d'un atome d'hydrogène,

sous réserve que seul l'un des substituants $R'_5$ à $R'_8$ représente une liaison covalente.

Parmi les composés de formule (I), on peut citer plus particulièrement l'isatine.

Les composés de formule (II) ou (III) préférentiels sont choisis parmi:

la 2,3-diaminopyridine
la 3,4-diaminopyridine
la 2-aminopyridine
la 5,6-diamino 2,4-dihydroxypyrimidine,
la 4,6-diaminopyrimidine,
la 2,6-diméthoxy 3,5-diaminopyridine, et
la 6-méthoxy 2,3-diaminopyridine.

En plus de ces composés préférés, les composés plus particulièrement préférés sont :

la 2,5-diaminopyridine, et
la 2,4,5,6-tétraaminopyrimidine.

Selon le procédé de la présente invention, les composés de formule (I) sont de préférence présents dans le composant (A) dans des proportions comprises entre 0,01 et 5% en poids et plus particulièrement entre 0,25 et 2% en poids par rapport au poids total du composant (A) ou des composants (A) + (B) et les composés de formule (II) ou (III) sont présents dans le composant (B) dans des proportions comprises de préférence entre 0,01 et 5% en poids et en particulier entre 0,25 et 2% en poids par rapport au poids total du composant (B) ou des composants (A) + (B).

Les composants (A) et (B) utilisables conformément à l'invention, sont des compositions liquides plus ou moins épaissies, aqueuses ou anhydres, des crèmes, des gels aqueux ou anhydres, des huiles ou des poudres à diluer avec un liquide au moment de l'emploi, encore appelées "cataplasmes".

Dans une première forme de réalisation de l'invention, le milieu cosmétique approprié pour la teinture est aqueux et a un pH pouvant varier entre 2 et 10, et de préférence entre 3 et 9,5, il est ajusté à la valeur désirée à l'aide d'agents alcalinisants ou d'agents acidifiants connus en eux-mêmes.

Ces compositions peuvent contenir des agents tensio-actifs anioniques, cationiques, non-ioniques, amphotères ou leurs mélanges. Ces tensio-actifs sont présents dans les compositions conformes à l'invention dans des proportions comprises entre 0,1 et 55% en poids, et de préférence entre 1 et 40% en poids par rapport au poids total de chaque composition.

Ces compositions aqueuses peuvent renfermer des solvants organiques parmi lesquels on peut mentionner à titre d'exemple, les alcanols inférieurs tels que l'éthanol ou l'isopropanol, les polyols tels que le glycérol, les glycols ou éthers de glycols comme l'éthylène glycol, le propylèneglycol, l'éther monobutylique de l'éthylèneglycol, le monoéthyléther et le monométhyléther de diéthylèneglycol, ainsi que des produits analogues ou leurs mélanges.

Ces solvants sont de préférence utilisés dans des proportions allant de 1 à 60% en poids, et plus particulièrement de 3 à 30% en poids par rapport au poids total de chaque composition.

Ces compositions peuvent être épaissies avec des agents choisis parmi l'alginate de sodium, la gomme arabique, la gomme de guar ou de caroube, la gomme de xanthane, les scléroglucanes, les pectines, les dérivés de la cellulose et les polymères divers ayant une fonction épaississante tels que les dérivés d'acide acrylique. On peut également utiliser des agents épaississants minéraux tels que la bentonite.

Ces agents épaississants sont présents de préférence dans des proportions comprises entre 0,1 et 5% en poids et en particulier entre 0,5 et 3% en poids par rapport au poids total de chaque composition.

Ces compositions peuvent également contenir des polymères anioniques, non-ioniques, cationiques, amphotères ou leurs mélanges, en des proportions de 0,1 à 5% en poids par rapport au poids total de chaque composition.

Ces compositions peuvent bien entendu contenir tous autres adjuvants habituellement utilisés dans les compositions pour la teinture des cheveux, tels que les agents de pénétration, les agents séquestrants, les agents antioxydants, des tampons, des parfums, des colorants, etc...

Une forme préférée de l'invention consiste à utiliser un milieu anhydre tel que décrit dans le brevet français n° 2.526.031.

On entend par milieu anhydre un milieu ne contenant pas plus de 1% d'eau.

Le milieu anhydre est constitué conformément à cette variante de l'invention, par un mélange d'au moins un solvant anhydre et d'un ou plusieurs agents tensio-actifs anhydres, de telle sorte que ces compositions contiennent au moins 15% de solvant et au moins 20% d'agent tensio-actif.

Les solvants utilisés sont des solvants cosmétiquement acceptables choisis parmi les monoalcools saturés en $C_2$-$C_{20}$ tels que l'éthanol, l'isopropanol, l'alcool cétylique ou l'octyldodécanol; les polyols tels que les alcoylèneglycols comme l'éthylèneglycol, le propylèneglycol, le glycérol, le diéthylèneglycol; les éthers de glycols tels que les mono-, di- et triéthylèneglycolmonoalcoyléthers comme par exemple l'éthylèneglycolmonoéthyléther, l'éthylèneglycolmonobutyléther, le diéthylèneglycolmonoéthyléther; des esters comme par exemple l'acétate de monométhyléther de l'éthylèneglycol, l'acétate de monoéthyléther de l'éthylèneglycol; les esters d'acides gras et d'alcools inférieurs saturés comme le myristate ou le palmitate d'isopropyle.

Les compositions particulièrement préférées contienent un solvant choisi parmi l'éthanol, l'alcool cétylique, le propylèneglycol, l'éthylèneglycolmonoéthyléther ou l'éthylèneglycolmonobutyléther.

Les agents tensio-actifs utilisés dans cette forme de réalisation sont choisis parmi les agents tensio-actifs anhydres de type anionique, non-ionique, cationique, amphotère ou leurs mélanges. On peut citer plus particulièrement les alcools gras polyoxyéthylénés, les alkylphénols ou naphtols polyoxyéthylénés, les halogénures de monoalkyltriméthylammonium, les halogénures de dialkyldiméthylammonium, les savons, les alcools gras polyglycérolés. Les agents tensio-actifs préférés sont les agents tensio-actifs non-ioniques.

Ces compositions peuvent contenir un agent alcalin ou acidifiant anhydre tel que par exemple l'acide citrique, l'acide ascorbique, l'acide acétique, l'acide lactique et des alcanolamines telles que, de préférence, celles qui sont totalement substituées sur le groupement amine comme le diméthylaminoéthanol.

En-dehors des composés décrits ci-dessus, les compositions anhydres conformes à l'invention peuvent contenir de nombreux additifs utilisables en cosmétique à la seule condition qu'ils contiennent moins de 1% d'eau. Parmi ces additifs, on peut citer les parfums, les agents épaississants, les agents traitants, les agents antioxydants, les huiles végétales ou minérales, les agents conservateurs et les sels organiques.

Ces compositions peuvent être appliquées telles quelles sur les cheveux mouillés ou être diluées tout juste avant l'emploi. Dans ce dernier cas, au moment de la teinture, les compositions selon l'invention sont diluées avec une solution aqueuse, de telle sorte que le rapport entre la composition conforme à l'invention et la solution aqueuse soit compris entre 0,25 et 2. La solution aqueuse peut être constituée par de l'eau pure, mais également par tout autre liquide aqueux complexe plus ou moins épaissi tel que par exemple un support habituellement utilisé dans les compositions tinctoriales pour cheveux.

Dans ce cas, les composants du milieu cosmétique peuvent être tous types d'ingrédients cosmétiquement acceptables, anhydres ou non, habituellement utilisés dans ce type de composition et décrits de façon générale ci-dessus.

Une autre forme d'utilisation des composants (A) et/ou (B) conformes à l'invention, est constituée par l'utilisation sous forme de cataplasmes, c'est-à-dire sous forme de poudre à diluer avec un liquide au moment de l'emploi.

Dans cette forme de réalisation, les colorants sont préparés sous forme de poudre stable au stockage et introduits dans un milieu solide pouvant être constitué de poudres, de farines, de substances amylacées ou mucilagineuses que l'on dilue au moment de l'emploi avec un liquide adéquat de façon à former un mélange ayant une consistance appropriée pour être appliqué sur tête.

Les poudres ou farines utilisées dans ce type de composition sont constituées généralement par des substances insolubles telles que des silices, des argiles, des végétaux pulvérisés après extraction de leurs principes actifs par solvant.

Le liquide peut être constitué par de l'eau ou des mélanges d'eau et de solvants cosmétiquement acceptables tels que des alcools ou des glycols ou encore par des huiles.

Le milieu liquide est additionné à la poudre dans des proportions telles qu'après mélange, on obtienne une pâte ayant une viscosité comprise entre 0,3 et 5 Pa.s.

Un objet de l'invention est constitué par un agent de teinture pour les fibres kératiniques, en particulier des cheveux humains, caractérisé par le fait qu'il est constitué par les composants (A) et (B) stockés sous forme séparée, tels que définis ci-dessus.

Les composants (A) et (B) sont destinés, soit à être mélangés tout juste avant emploi, soit à être appliqués de façon successive sur les fibres à traiter.

Selon une forme de réalisation, on peut conditionner les différents composants (A) et (B) dans un dispositif à plusieurs compartiments encore appelé "kit de teinture" comportant tous les composants destinés à être appliqués pour une même teinture sur les fibres kératiniques, en particulier les cheveux, en applications successives avec ou sans prémélange.

De tels dispositifs peuvent comporter un premier compartiment contenant le composant (A) renfermant l'isatine ou ses dérivés de formule (I) et un second compartiment comportant le composant (B) renfermant l'aminopyridine de formule (II) ou l'aminopyrimidine de formule (III).

Une autre variante peut également consister à stocker le composant (A) ou le composant (B) dans un milieu solvant anhydre et à prévoir un troisième compartiment contenant un milieu aqueux approprié pour la teinture et cosmétiquement acceptable. Dans ce cas, on mélange tout juste avant l'emploi le contenu du troisième compartiment dans l'un ou l'autre ou les deux compartiments contenant les composants anhydres (A) et (B) ou alors on mélange avant emploi les trois compartiments.

Selon une variante, le procédé de l'invention consiste à mélanger juste avant l'emploi le composant (A) au composant (B), la composition résultante étant appliquée sur les cheveux pendant 5 à 40 minutes et de préférence 20 à 30 minutes. Les cheveux sont alors rincés, lavés au shampooing, rincés à nouveau puis séchés.

Selon une autre variante, le procédé de l'invention consiste à appliquer sur les cheveux au moins un composant (A) et, avant ou après le composant (A), un composant (B) tels que définis ci-dessus; à laisser poser chacun d'entre eux pendant 5 à 40 minutes, de préférence 20 à 30 minutes, en rinçant éventuellement à l'eau entre les deux étapes. Les cheveux sont ensuite rincés, lavés au shampooing, rincés à nouveau puis séchés.

Les exemples suivants sont destinés à illustrer l'invention sans pour autant présenter un caractère limitatif.

EXEMPLES 1 à 4

On procède à la teinture des cheveux en appliquant sur des cheveux naturels gris à 90% de blancs, 20 g des compositions.

Les compositions sont préparées juste avant l'emploi.

On laisse agir la composition pendant 20 minutes, puis on rince les cheveux, on effectue un shampooing puis on rince à nouveau. Après séchage, les cheveux sont teints dans la nuance précisée au bas du tableau I ci-après.

TABLEAU I

| en g MA | 1 | 2 | 3 | 4 |
|---|---|---|---|---|
| Isatine | 1 | 1 | 0,5 | 1 |
| Tétraaminopyrimidine | 1 | | 0,5 | |
| 2,5-diaminopyridine | | 1 | | |
| 2-aminopyridine | | | | 1 |
| Alcool éthylique | 30 | 30 | 30 | 30 |
| Lauryléthersulfate de sodium oxyéthyléné à 2 moles d'oxyde d'éthylène à 28% de MA | | | 8,4 | |
| Triéthanolamine qs pH | 7,6 | 7,3 | 8 | 8,6 |
| Eau qsp | 100 | 100 | 100 | 100 |
| **Nuances obtenues** | rouge cuivré | marron cuivré | irisé cuivré | cuivré doré intense |

EXEMPLES 5 à 7

On procède à la teinture des cheveux en appliquant sur des cheveux permanentés gris à 90% de blancs, 20 g des compositions.

Les compositions sont préparées juste avant l'emploi.

On laisse agir la composition pendant 20 minutes, puis on rince les cheveux (dans l'exemple 7, on effectue ensuite un shampooing puis on rince à nouveau). Après séchage, les cheveux sont teints dans la nuance précisée au bas du tableau II ci-après.

EXEMPLE 8

On procède à la teinture de cheveux permanentés gris à 90% de blancs.

On applique 5 g de la composition A sur 3 g de cheveux pendant 15 minutes, la composition à étant diluée avant l'emploi avec 3 fois son poids en eau.

Après un rinçage des cheveux, on applique 8 g de la composiiton B pendant 15 minutes, la composition B étant diluée avec 1,5 fois son poids en eau avant l'emploi.

Les cheveux sont ensutie rincés puis séchés.

On obtient en final une coloration dont la nuance est indiquée dans le tableau II ci-après.

TABLEAU II

| en g MA | 5 | 6 | 7 | 8 | |
|---|---|---|---|---|---|
| | | | | Comp. A | Comp. B |
| Isatine | 1 | 1 | | 4 | |
| 5-chloroisatine | | | 0,25 | | |
| 2,6-diméthoxy 3,5-diamino-pyridine | 1 | | | | |
| 6-méthoxy 2,3-diamino-pyridine | | 1 | 0,25 | | 1 |
| Gomme de caroube vendue sous la dénomination Vidogum L 175 par la Société Sanofi Bio Industrie | | | | 3 | |
| Carbonate de calcium | | | | 8 | |
| Poudre de résidus d'épuisement de saponaire de granulométrie inférieure à 90 microns | | | | 35 | |
| Lait écrémé en poudre qsp | | | | 100 | |
| Alcool éthylique | 30 | 30 | 30 | | 28,5 |
| Lauryléthersulfate de sodium oxyéthylèné à 2 moles d'oxyde d'éthylène à 28% de MA | | | 8,4 | | |
| Triéthanolamine | | | | | 1 |
| Nonylphénol oxyéthylèné à 9 moles d'oxyde d'éthylène qsp | | | | | 100 |
| Triéthanolamine qs pH | 8 | 8 | 8 | | |
| Eau qsp | 100 | 100 | 100 | | |
| **Nuances obtenues** | blond mat doré | rouge puissant | blond clair irisé | blond très clair irisé | |

## Revendications

1. Procédé de teinture des fibres kératiniques, en particulier des cheveux humains, caractérisé par le fait que l'on applique sur lesdites fibres un composant (A) constitué d'une composition renfermant dans un milieu approprié pour la teinture, au moins un composé de formule (I) :

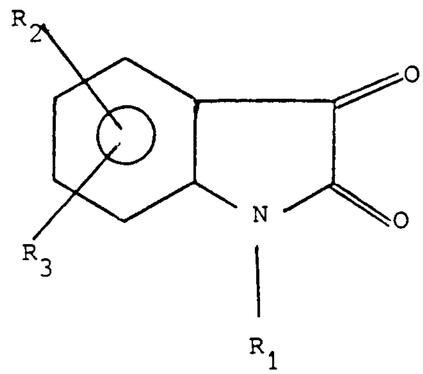

(I)

dans laquelle :

$R_1$ désigne un atome d'hydrogène, un radical allyle en $C_1$-$C_6$, acétyle, benzoyle, phényle ou carboxyalkyle en $C_1$-$C_4$;

$R_2$ et $R_3$, indépendamment l'un de l'autre, désignent un atome d'hydrogène, un alkyle en $C_1$-$C_6$, un hydroxyle, un atome d'halogène, un groupement nitro, un alkyl($C_1$-$C_6$)phényle, phényle ou un alcoxy en $C_1$-$C_4$;

et un composant (B) constitué d'une composition contenant dans un milieu approprié pour la teinture, au moins une aminopyridine de formule (II) suivante :

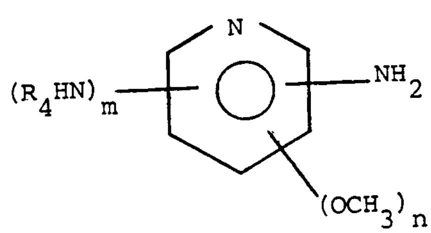

(II)

dans laquelle :

$R_4$ désigne un atome d'hydrogène ou un groupement $\beta$-hydroxyéthyle;

n = O, 1 ou 2, et m = O ou 1;

ainsi que ses sels cosmétiquement acceptables,

ou bien une aminopyrimidine de formule (III) :

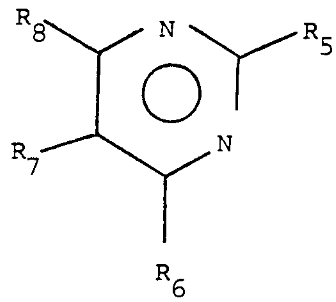

(III)

dans laquelle :

$R_5$ désigne un atome d'hydrogène, un groupement hydroxy ou un groupement

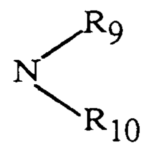

$R_6$ désigne un groupement hydroxy ou un groupement

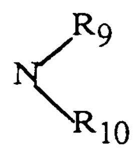

$R_7$ désigne H ou $NH_2$,

$R_8$ désigne un groupement

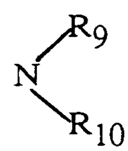

$R_9$ et $R_{10}$, indépendamment l'un de l'autre, représentant un atome d'hydrogène, un allyle en $C_1$-$C_4$, un groupement $(CH_2)_p$-Z, où p = 1 à 4 et Z représente OH, halogène, $NH_2$, NHR′, ou NHR′R″, où R′et R″ désignent un allyle en $C_1$-$C_4$ ou forment un hétérocycle avec l'atome d'azote auxquels ils sont attachés ;

sous réserve qu'un des groupements $R_5$ à $R_8$ désigne $NH_2$;

ainsi que ses sels cosmétiquement acceptables.

**2.** Procédé selon la revendication 1, caractérisé par le fait que le composé de formule (I) est l'isatine.

**3.** Procédé selon la revendication 1 ou 2, caractérisé par le fait que le composé de formule (II) ou (III) est choisi parmi :

la 2,3-diaminopyridine
la 3,4-diaminopyridine
la 2-aminopyridine
la 5,6-diamino 2,4-dihydroxypyrimidine,
la 4,6-diaminopyrimidine,
la 2,6-diméthoxy 3,5-diaminopyridine, et
la 6-méthoxy 2,3-diaminopyridine,

**4.** Procédé selon la revendication 1 ou 2, caractérisé par le fait que le composé de formule (II) est la 2,5-diaminopyridine ou le composé de formule (III) est la 2,4,5,6-tétraaminopyrimidine.

**5.** Procédé selon l'une quelconque des revendications 1 à 4, caractérisé par le fait que les composés de formule (I) sont présents dans le composant (A) dans des proportions comprises entre 0,01 et 5% en poids par rapport au poids du composant (A) ou au poids total de (A) + (B) et que les composés de formule (II) ou (III) sont présents dans le composant (B) dans des proportions comprises entre 0,01 et 5% en poids par rapport au poids du composant (B) ou au poids total de (A) + (B).

**6.** Procédé selon l'une quelconque des revendications 1 à 5, caractérisé par le fait que le composant (A) et/ou le composant (B) est une composition aqueuse ou anhydre sous forme liquide plus ou moins

épaissie, une composition sous forme de crème, de gel aqueux ou anhydre, d'huile ou de poudre à diluer avec un liquide au moment de l'emploi.

**7.** Procédé selon la revendication 6, caractérisé par le fait que le composant (A) et/ou le composant (B) se présente(nt) sous forme d'une composition aqueuse ayant un pH compris entre 2 et 10 et contenant un ou plusieurs adjuvants cosmétiquement acceptables, choisis parmi les agents tensio-actifs anioniques, cationiques, non-ioniques ou leurs mélanges, des solvants organiques, des polymères anioniques, nonioniques, cationiques, amphotères ou leurs mélanges, des agents épaississants, des agents de pénétration, des agents séquestrants, des agents antioxydants, des tampons, des colorants, des parfums.

**8.** Procédé selon la revendication 6, caractérisé par le fait que le composant (A) et/ou le composant (B) se présente(nt) sous forme d'une composition anhydre contenant un ou plusieurs solvants anhydres et un ou plusieurs tensio-actifs anhydres, dans des proportions d'au moins 15% de solvant et d'au moins 20% d'agent tensio-actif.

**9.** Procédé selon la revendication 8, caractérisé par le fait que le solvant anhydre est choisi parmi les monoalcools saturés en $C_2$-$C_{20}$, les polyols, les éthers de glycol, les esters de glycol, les esters d'acides gras et d'alcools inférieurs.

**10.** Procédé selon la revendication 6, caractérisé par le fait que le composant (A) et/ou le composant (B) se présente(nt) sous forme de poudre, à diluer avec un liquide au moment de l'emploi, constituée par des substances amylacées ou mucilagineuses ou par des poudres ou farines choisies parmi les silices, les argiles, les végétaux pulvérisés après extraction de leurs principes actifs par des solvants.

**11.** Procédé selon la revendication 10, caractérisé par le fait que l'on réalise un cataplasme à partir du composant (A) et/ou du composant (B) sous forme de poudre, par addition d'un liquide cosmétiquement acceptable, dans des proportions suffisantes pour obtenir une viscosité de 0,3 à 5 Pa.s.

**12.** Procédé selon l'une quelconque des revendications 1 à 11, caractérisé par le fait que l'on mélange les composants (A) et (B) juste avant emploi, que l'on applique immédiatement la composition résultante sur les fibres kératiniques, qu'on laisse agir pendant 5 à 40 minutes; les fibres kératiniques étant ensuite rincées, lavées au shampooing, rincées à nouveau, puis séchées.

**13.** Procédé selon l'une quelconque des revendications 1 à 11, caractérisé par le fait qu'il comporte l'application sur les fibres Kératiniques du composant (A) suivie ou précédée de l'application sur lesdites fibres du composant (B), que l'on laisse agir chaque composant pendant 5 à 40 minutes, que l'on procède éventuellement à un rinçage à l'eau entre chaque application; les fibres kératiniques étant ensuite rincées, lavées au shampooing, rincées à nouveau, puis séchées.

**14.** Agent de teinture des fibres kératiniques et en particulier des cheveux, caractérisé par le fait qu'il comporte les composants (A) et (B) tels que définis dans les revendications 1 à 11, sous forme séparée; les composants (A) et (B) étant destinés à être, soit mélangés tout juste avant emploi, soit appliqués de façon successive sur les fibres à traiter.

**15.** Dispositif à plusieurs compartiments ou "kit de teinture", caractérisé par le fait qu'il comporte au moins deux compartiments dont un renferme le composant (A) tel que défini dans l'une quelconque des revendications 1, 2, 5 à 11, et le second renferme le composant (B) tel que défini dans l'une quelconque des revendications 1, 3 à 11.

**16.** Dispositif selon la revendication 15, caractérisé par le fait que le composant (A) et/ou le composant (B) se présente(nt) sous forme de composition anhydre et qu'il comporte un troisième compartiment contenant un milieu aqueux cosmétiquement acceptable approprié pour la teinture destiné à être mélangé avant emploi dans l'un ou les deux premiers compartiments renfermant chaque composant (A) ou (B).

## Claims

1. Process for dyeing keratinous fibres, especially human hair, characterised in that there are applied on the said fibres a component (A) consisting of a composition containing, in a medium suitable for dyeing, at least one compound of formula (I):

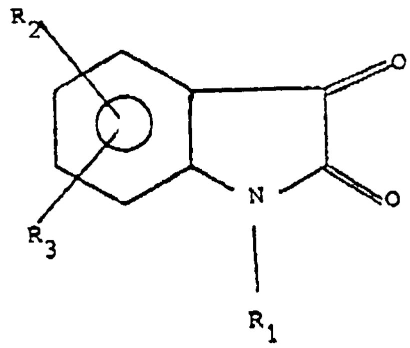

(I)

in which:

$R_1$ denotes a hydrogen atom or a $C_1$-$C_6$ alkyl, acetyl, benzoyl, phenyl or $C_1$-$C_4$ carboxyalkyl radical;

$R_2$ and $R_3$, independently of one another, denote a hydrogen atom, a $C_1$-$C_6$ alkyl, a hydroxyl, a halogen atom, a nitro group, a ($C_1$-$C_6$ alkyl)phenyl, phenyl or a $C_1$-$C_4$ alkoxy;

and a component (B) consisting of a composition containing, in a medium suitable for dyeing, at least one aminopyridine of the following formula (II):

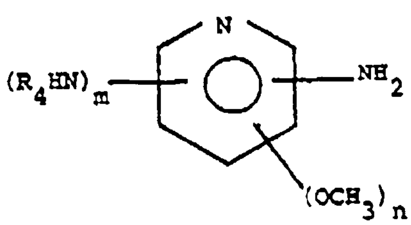

(II)

in which:

$R_4$ denotes a hydrogen atom or a $\beta$-hydroxyethyl group;

n = 0, 1 or 2, and m = 0 or 1;

as well as its cosmetically acceptable salts,

or alternatively one aminopyrimidine of formula (III):

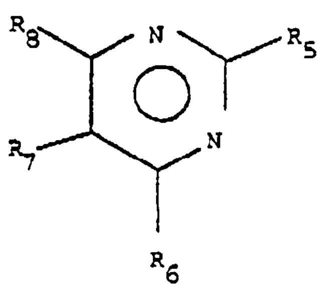

(III)

in which:

$R_5$ denotes a hydrogen atom, a hydroxyl group or a group

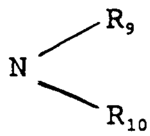

$R_6$ denotes a hydroxyl group or a group

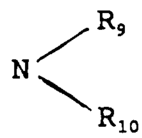

$R_7$ denotes H or $NH_2$,
$R_8$ denotes a group

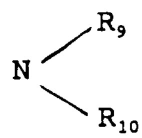

$R_9$ and $R_{10}$, independently of one another, representing a hydrogen atom, a $C_1$-$C_4$ alkyl, a group $(CH_2)_p$-Z, where p = 1 to 4 and Z represents OH, halogen, $NH_2$, NHR' or NHR'R'', where R' and R'' denote a $C_1$-$C_4$ alkyl or form a heterocycle with the nitrogen atom to which they are attached;
with the proviso that one of the groups $R_5$ to $R_8$ denotes $NH_2$;
as well as its cosmetically acceptable salts.

2. Process according to Claim 1, characterised in that the compound of formula (I) is isatin.

3. Process according to Claim 1 or 2, characterised in that the compound of formula (II) or (III) is chosen from:
   2,3-diaminopyridine
   3,4-diaminopyridine
   2-aminopyridine
   5,6-diamino-2,4-dihydroxypyrimidine,
   4,6-diaminopyrimidine,
   2,6-dimethoxy-3,5-diaminopyridine, and
   6-methoxy-2,3-diaminopyridine.

4. Process according to Claim 1 or 2, characterised in that the compound of formula (II) is 2,5-diaminopyridine or the compound of formula (III) is 2,4,5,6-tetraaminopyrimidine.

5. Process according to any one of Claims 1 to 4, characterised in that the compounds of formula (I) are present in the component (A) in proportions of between 0.01 and 5% by weight relative to the weight of the component (A) or to the total weight of (A) + (B), and in that the compounds of formula (II) or (III) are present in the component (B) in proportions of between 0.01 and 5% by weight relative to the weight of the component (B) or to the total weight of (A) + (B).

6. Process according to any one of Claims 1 to 5, characterised in that the component (A) and/or the component (B) is an aqueous or anhydrous composition in more or less thickened liquid form or a composition in the form of a cream, aqueous or anhydrous gel, oil or powder to be diluted with a liquid at the time of use.

7. Process according to Claim 6, characterised in that the component (A) and/or the component (B) take-(s) the form of an aqueous composition having a pH of between 2 and 10 and containing one or more

cosmetically acceptable adjuvants chosen from anionic, cationic and nonionic surfactants or mixtures thereof, organic solvents, anionic, nonionic, cationic and amphoteric polymers or mixtures thereof, thickening agents, penetrating agents, sequestering agents, antioxidants, buffers, dyes and perfumes.

**8.** Process according to Claim 6, characterised in that the component (A) and/or the component (B) take-(s) the form of an anhydrous composition containing one or more anhydrous solvents and one or more anhydrous surfactants, in proportions of at least 15% of solvent and at least 20% of surfactant.

**9.** Process according to Claim 8, characterised in that the anhydrous solvent is chosen from saturated $C_2$-$C_{20}$ monohydric alcohols, polyols, glycol ethers, glycol esters and esters of fatty acids and lower alcohols.

**10.** Process according to Claim 6, characterised in that the component (A) and/or the component (B) take-(s) the form of powder, to be diluted with a liquid at the time of use, consisting of amylaceous or mucilaginous substances or of powders or flours chosen from silicas, clays and plants powdered after solvent-extraction of their active principles.

**11.** Process according to Claim 10, characterised in that a cataplasm is made from the component (A) and/or the component (B) in powder form, by adding a cosmetically acceptable liquid in proportions sufficient to obtain a viscosity of 0.3 to 5 Pa.s.

**12.** Process according to any one of Claims 1 to 11, characterised in that the components (A) and (B) are mixed immediately before use, in that the resulting composition is applied immediately on the keratinous fibres and in that it is left to act for 5 to 40 minutes; the keratinous fibres then being rinsed, washed with shampoo, rinsed again and thereafter dried.

**13.** Process according to any one of Claims 1 to 11, characterised in that it entails the application of the component (A) on the keratinous fibres followed or preceded by the application of the component (B) on the said fibres, in that each component is left to act for 5 to 40 minutes and in that a rinse with water is optionally performed between each application; the keratinous fibres then being rinsed, washed with shampoo, rinsed again and thereafter dried.

**14.** Dyeing agent for keratinous fibres, and especially hair, characterised in that it contains the components (A) and (B) as defined in Claims 1 to 11, in separate form; the components (A) and (B) being designed to be either mixed at the very last moment before use, or applied successively on the fibres to be treated.

**15.** Multi-compartment device or "dyeing kit", characterised in that it possesses at least two compartments, one of which contains the component (A) as defined in any one of Claims 1, 2 and 5 to 11, and the other contains the component (B) as defined in any one of Claims 1 and 3 to 11.

**16.** Device according to Claim 15, characterised in that the component (A) and/or the component (B) take-(s) the form of an anhydrous composition, and in that it possesses a third compartment containing a cosmetically acceptable aqueous medium suitable for dyeing, designed to be mixed before use into one or both first compartments containing each component (A) or (B).

**Patentansprüche**

**1.** Verfahren zur Färbung keratinischer Fasern, insbesondere menschlicher Haare,
dadurch **gekennzeichnet**, daß
man auf die genannten Fasern einen Bestandteil (A) aus einer Zusammensetzung, die in einem zur Färbung geeigneten Milieu mindestens eine Verbindung der folgenden Formel (I) enthält:

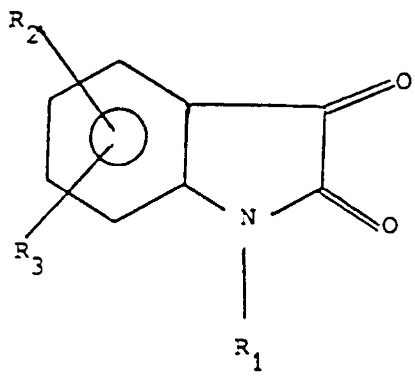

(I)

worin gilt:
$R_1$ bedeutet ein Wasserstoffatom, einen $C_{1-6}$-Alkyl-, Acetyl-, Benzoyl-, Phenyl- oder $C_{1-4}$-Carboxyalkylrest,
$R_2$ und $R_3$ bedeuten, unabhängig voneinander, ein Wasserstoffatom, einen $C_{1-6}$-Alkylrest, einen Hydroxylrest, ein Halogenatom, eine Nitrogruppe, einen $C_{1-6}$-Alkylphenyl-, Phenyl- oder $C_{1-4}$-Alkoxyrest;
sowie einen Bestandteil (B) aus einer Zusammensetzung aufbringt, die in einem zur Färbung geeigneten Milieu mindestens
ein Aminopyridin der folgenden Formel (II):

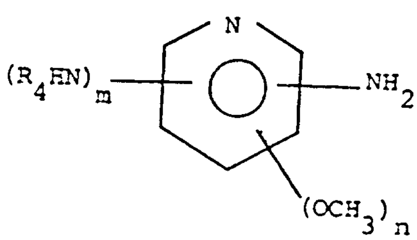

(II)

worin gilt:
$R_4$ bedeutet ein Wasserstoffatom oder eine $\beta$-Hydroxyethylgrupppe,
n = 0, 1 oder 2 und m = 0 oder 1;
sowie seine kosmetisch verträglichen Salze,
oder auch ein Aminopyrimidin der Formel (III):

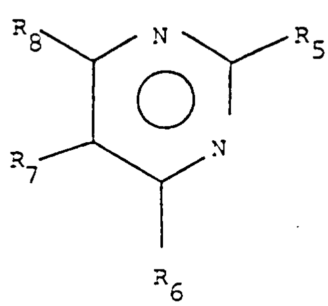

(III)

worin gilt:
$R_5$ bedeutet ein Wasserstoffatom, eine Hydroxygruppe oder eine Gruppe

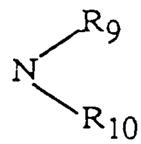

$R_6$ bedeutet eine Hydroxygruppe oder eine Gruppe

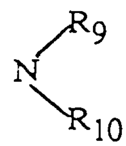

$R_7$ bedeutet H oder $NH_2$,
$R_8$ bedeutet eine Gruppe

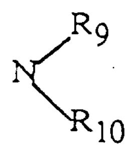

$R_9$ und $R_{10}$ stellen, unabhängig voneinander, ein Wasserstoffatom, einen $C_{1-4}$-Alkylrest, eine Gruppe $(CH_2)_p$-Z, worin p = 1 bis 4 und Z OH, Halogen, $NH_2$, NHR' oder NHR'R'' darstellt, worin R' und R'' einen $C_{1-4}$-Alkylrest bedeuten oder einen Heterozyklus mit dem Stickstoffatom bilden, an das sie gebunden sind;
mit der Maßgabe, daß eine der Gruppen $R_5$ bis $R_8$ $NH_2$ bedeutet;
sowie seine kosmetisch verträglichen Salze enthält.

**2.** Verfahren gemäß Anspruch 1,
dadurch **gekennzeichnet**, daß
die Verbindung der Formel (I) Isatin ist.

**3.** Verfahren gemäß Anspruch 1 oder 2,
dadurch **gekennzeichnet**, daß
die Verbindung der Formel (II) oder (III) ausgewählt ist aus: 2,3-Diaminopyridin, 3,4-Diaminopyridin, 2-Aminopyridin, 5,6-Diamino-2,4-dihydropyrimidin, 4,6-Diaminopyrimidin, 2,6-Dimethoxy-3,5-diaminopyridin und 6-Methoxy-2,3-diaminopyridin.

**4.** Verfahren gemäß Anspruch 1 oder 2,
dadurch **gekennzeichnet**, daß
die Verbindung der Formel (II) 2,5-Diaminopyrimidin oder die Verbindung der Formel (III) 2,4,5,6-Tetraaminopyrimidin sind.

**5.** Verfahren gemäß jedem Anspruch 1 bis 4,
dadurch **gekennzeichnet**, daß
die Verbindungen der Formel (I) im Bestandteil (A) in Mengenanteilen von 0,01 bis 5 Gew.%, bezogen auf Gesamtgewicht des Bestandteils (A) oder auf das Gesamtgewicht von (A) + (B), und die Verbindungen der Formel (II) oder (III) im Bestandteil (B) in Mengenanteilen von 0,01 bis 5 Gew.% vorhanden sind, bezogen auf das Gewicht des Bestandteils (B) oder auf das Gesamtgewicht von (A) + (B).

**6.** Verfahren gemäß jedem Anspruch 1 bis 5,
dadurch **gekennzeichnet**, daß

der Bestandteil (A) und/oder der Bestandteil (B) eine wässrige oder wasserfreie Zusammensetzung in mehr oder weniger verdickter flüssiger Form, eine Zusammensetzung in Form einer Creme, eines wässrigen oder wasserfreien Gels, eines Öls oder Pulvers zur Verdünnung mit einer Flüssigkeit zum Zeitpunkt des Gebrauchs sind.

**7.** Verfahren gemäß Anspruch 6,
dadurch **gekennzeichnet**, daß
der Bestandteil (A) und/oder der Bestandteil (B) in Form einer wässrigen Zusammensetzung mit einem pH-Wert von 2 bis 10 vorliegen und einen oder mehrere kosmetisch verträgliche Hilfsstoffe enthalten, ausgewählt aus anionischen, kationischen, nicht-ionischen oberflächenaktiven Mitteln oder deren Mischungen, aus organischen Lösungsmitteln, anionischen, nicht-ionischen, kationischen oder amphoteren Polymeren oder aus deren Mischungen, aus Verdickungsmitteln, Eindringmitteln, Sequestriermitteln, Antioxidantien, Puffermitteln, Farbstoffen und Parfüm-Produkten.

**8.** Verfahren gemäß Anspruch 6,
dadurch **gekennzeichnet**, daß
der Bestandteil (A) und/oder der Bestandteil (B) in Form einer wasserfreien Zusammensetzung vorliegen, welche ein oder mehrere wasserfreie Lösungsmittel und ein oder mehrere wasserfreie oberflächenaktive Mittel in Mengenanteilen von mindestens 15% Lösungsmittel und mindestens 20% oberflächenaktivem Mittel enthalten.

**9.** Verfahren gemäß Anspruch 8,
dadurch **gekennzeichnet**, daß
das wasserfreie Lösungsmittel aus gesättigten $C_{2-20}$-Monoalkoholen, Polyolen, Glycolethern, Glycolestern, Estern von Fettsäuren und Niedrigalkoholen ausgewählt ist.

**10.** Verfahren gemäß Anspruch 6,
dadurch **gekennzeichnet**, daß
der Bestandteil (A) und/oder der Bestandteil (B) in Form eines Pulvers zur Verdünnung mit einer Flüssigkeit zum Zeitpunkt des Gebrauchs aus Stärke- oder Pflanzenschleimsubstanzen oder aus Pulvern oder aus Mehlen aus Kieselsäuren, Tonen oder pflanzlichen Substanzen, die nach einer mit Lösungsmitteln durchgeführten Extraktion ihrer Wirkstoffe pulverisiert sind, vorliegen.

**11.** Verfahren gemäß Anspruch 10,
dadurch **gekennzeichnet**, daß
man ein Kataplasma aus dem Bestandteil (A) und/oder dem Bestandteil (B) in Form eines Pulvers durch Zugabe einer kosmetisch verträglichen Flüssigkeit in Mengenanteilen herstellt, die ausreichen, um eine Viskosität von 0,3 bis 5 Pa x s zu erhalten.

**12.** Verfahren gemäß jedem Anspruch 1 bis 11,
dadurch **gekennzeichnet**, daß
man die Bestandteile (A) und (B) kurz vor Gebrauch vermischt, die sich ergebende Zusammensetzung sofort auf die keratinischen Fasern aufbringt und das Ganze 5 bis 40 Minuten lang einwirken läßt, worauf die keratinischen Fasern gespült, unter Schamponieren gewaschen, erneut gespült und dann getrocknet werden.

**13.** Verfahren gemäß jedem Anspruch 1 bis 11,
dadurch **gekennzeichnet**, daß
man auf die keratinischen Fasern den Bestandteil (A) vor oder nach der Aufbringung des Bestandteils (B) aufträgt, jeden Bestandteil 5 bis 40 Minuten lang einwirken läßt und gegebenenfalls zwischen jeder Aufbringung mit Wasser spült, worauf die keratinischen Fasern gespült, unter Schamponieren erneut gewaschen, erneut gespült und dann getrocknet werden.

**14.** Mittel zur Färbung keratinischer Fasern und insbesondere der Haare,
dadurch **gekennzeichnet**, daß
es die in den Ansprüchen 1 bis 11 definierten Bestandteile (A) und (B) in getrennter Form enthält, wobei die Bestandteile (A) und (B) dazu bestimmt sind, entweder kurz vor Gebrauch vermischt oder nacheinander auf die zu behandelnden Fasern aufgebracht zu werden.

**15.** Vorrichtung aus mehreren Teilen oder "Kit zur Färbung", dadurch **gekennzeichnet**, daß sie mindestens 2 Teile aufweisen, wovon der eine den in jedem der Ansprüche 1, 2 und 5 bis 11 definierten Bestandteil (A) und der zweite den in einem jeden der Ansprüche 1 und 3 bis 11 definierten Bestandteil (B) umfassen.

**16.** Vorrichtung gemäß Anspruch 15, dadurch **gekennzeichnet**, daß der Bestandteil (A) und/oder der Bestandteil (B) in Form einer wasserfreien Zusammensetzung vorliegen, und daß die Vorrichtung einen dritten Teil aufweist, der ein zur Färbung geeignetes, kosmetisch verträgliches wässriges Milieu enthält, das dazu bestimmt ist, vor Gebrauch in dem einen oder den beiden ersten Teilen vermischt zu werden, welche jeden Bestandteil (A) oder (B) aufweisen.